# EUROPEAN PATENT APPLICATION

(11) **EP 4 464 777 A1**
(43) Date of publication of application: **20.11.2024**
(21) Application number: 23740534.5
(22) Date of filing: 16.01.2023
(51) Int. Cl.: C12N 5/0775, C12M 1/34, C12M 1/00, C12N 5/00

(54) **METHOD FOR INCREASING EXOSOME PRODUCTIVITY AND COMPOSITION COMPRISING EXOSOMES PRODUCED THEREBY**

(30) Priority: 14.01.2022 KR 20220005693; 05.07.2022 KR 20220082798; 08.08.2022 KR 20220098866
(71) Applicant: Kim, Seung Chan, Seongnam-si, Gyeonggi-do 13621 (KR)
(72) Inventor: Kim, Seung Chan, Seongnam-si, Gyeonggi-do 13621 (KR)
(74) Representative: Müller Schupfner & Partner Patent- und Rechtsanwaltspartnerschaft mbB (Muc)
(86) International application number: PCT/KR2023/000753
(87) International publication number: WO 2023/136691

(57) **Abstract**

The present invention relates to a method for improving exosome productivity by treatment using high-pressure oxygen, and a composition comprising exosomes produced thereby, and when the method of the present invention is used, the exosome productivity can be effectively improved, which can be very useful for diagnosis and treatment using exosomes.

## Description

### Technical Field

The present invention relates to a method for increasing exosome productivity using high-pressure oxygen treatment and a composition comprising exosomes produced thereby.

### Background Art

Exosomes are small membrane-structured vesicles secreted from various types of cells. The diameters of exosomes are reported to be approximately 30 to 300 nm. The exosomes have been observed in electron microscopy studies to originate from specific compartments within cells called multivesicular bodies (MVBs) and to be released and secreted outside the cells, rather than being detached directly from the plasma membrane. That is, when fusion of the MVBs and the plasma membrane occurs, the vesicles are released into an extracellular environment, which are called exosomes. Although a molecular mechanism by which these exosomes are produced has not been clearly found, but it is known that not only red blood cells, but also various types of immune cells including B lymphocytes, T lymphocytes, dendritic cells, platelets, macrophages, tumor cells, stem cells, etc. produce and secrete exosomes while alive.

In addition, according to recent studies, it has been reported that exosomes play an important role in processes such as intercellular signaling and disposal management (Edwin van der pol et al., Pharmacological Reviews July 2012, 64 (3) 676-705). The exosomes have the potential to be used as biomarkers for prognosis on diseases, treatment, and health and disease, and recently, interest in clinical applications has been increased.

The background art of the invention has been prepared to more facilitate understanding of the present invention. It should not be understood that the matters described in the background art of the invention exist as prior arts.

### Disclosure

### Technical Problem

Meanwhile, since the amount of exosomes produced by cells is small, there is a need for a method capable of increasing exosome productivity to efficiently obtain a large amount of exosomes.

Accordingly, the present inventors intended to develop a method capable of effectively increasing exosome productivity.

As a result, the present inventors developed a method for increasing exosome productivity by treating high-pressure oxygen when stem cells are cultured.

An object of the present invention is to provide a method for increasing exosome productivity by treating high-pressure oxygen in a process of culturing stem cells.

The objects of the present invention are not limited to the aforementioned objects, and other objects, which are not mentioned above, will be apparent to those skilled in the art from the following description.

### Technical Solution

One aspect of the present invention provides a method including culturing a sample containing cells in a first pressure environment; and culturing the sample in a second pressure environment higher than the first pressure, in which the second pressure condition is created by high-pressure oxygen.

In the present invention, the "cells" may be all types of somatic cells obtained from the body, and for example, may be at least one selected from the group consisting of skin, hair follicles, tooth roots, fat, blood, urine, mucus, saliva, tears, plasma, serum, urine, sputum, spinal fluid, pleural effusion, nipple aspirate, lymph fluid, respiratory fluid, intestinal fluid, urogenital tract fluid, breast milk, lymphatic fluid, semen, cerebrospinal fluid, tracheal fluid, ascites, cystic tumor fluid, amniotic fluid, and combinations thereof, and are not limited thereto as long as the cells are cells that secrete exosomes. In addition, the "cells" may be cells obtained from organisms other than humans, such as plants or animals, in addition to somatic cells obtained from the body.

In the present invention, the "exosome" means a small membrane vesicle having a lipid bilayer derived from an endosome released by a cell, and the exosome may refer to a small vesicle containing protein, DNA, RNA, etc. and secreted outside a cell to transmit signals between cells, and may include, for example, protein, lipid, mRNA, microRNA (miRNA), and genomic DNA.

In an exemplary embodiment, the stem cell-derived exosome may include a marker protein selected from the group consisting of CD9, CD63, CD81, and a combination thereof, but is not limited thereto.

Specifically, since CD9, CD63, and CD81 are representative positive markers of exosomes, the contents of CD9, CD63, and CD81 and the content of exosomes have linearity to each other, and an increase in the contents of CD9, CD63, and CD81 means a proportional increase in the content of exosomes.

In a specific exemplary embodiment, the exosomes are not limited, but may be derived, extracted, or isolated from fat or human blood. Alternatively, the exosomes may be extracted from stem cells or immune cells. The stem cells may be embryonic stem cells, induced pluripotent stem cells (iPSCs), adult stem cells, embryonic stem cell-derived mesenchymal stem cells, or induced pluripotent stem cell-derived mesenchymal stem cells. The immune cells may be T cells, B cells, NK cells, cytotoxic T cells, dendritic cells or macrophages. In addition, the exosomes may be derived, extracted, or isolated from cells obtained from organisms other than humans, such as plants or animals, in addition to cells obtained from the body.

In addition, as an example that does not limit the present invention, the adult stem cells may be one or more adult stem cells selected from the group consisting of mesenchymal stem cells, human tissue-derived mesenchymal stromal cells, human tissue-derived mesenchymal stem cells, and multipotent stem cells. The mesenchymal stem cells may be mesenchymal stem cells derived from one or more tissues selected from the group consisting of umbilical cord, umbilical cord blood, bone marrow, fat, muscle, nerve, skin, amniotic membrane, Wharton's jelly and placenta. Preferably, the adult stem cells may be mesenchymal stem cells, for example, stem cells derived from fat, bone marrow, umbilical cord or umbilical cord blood, and more preferably, stem cells derived from fat.

In a specific exemplary embodiment, when the exosomes are extracted from stem cells, the stem cells may be stem cells (e.g., human adipose-derived stem cells) isolated from adipose tissue (e.g., human adipose tissue), but are not limited thereto, and may include all cells capable of differentiating into one or more mesenchymal cells, such as fat, bone, cartilage, and myofiber. The adipose-derived stem cells (ADSCs) may be at least one type of preadipocyte cells, stromal cells, multipotent adipose-derived cells, or adipose-derived adult stem cells.

The type of stem cell or immune cell is not limited as long as there is no risk of infection by a pathogen and no immune rejection response is caused, but may be preferably a human-derived stem cell or a human-derived immune cell.

However, it should be understood that the cells may include various animal cells and plant cells that have been used or may be used later in the art, and the adipose stem cells used in examples to be described below are one example of animal cells that may be used in the present invention, but are not limited thereto.

In the present invention, the "sample" may be at least one selected from the group consisting of blood, urine, mucus, saliva, tears, plasma, serum, urine, sputum, spinal fluid, pleural effusion, nipple aspirate, lymph fluid, respiratory fluid, intestinal fluid, urogenital tract fluid, breast milk, lymphatic fluid, semen, cerebrospinal fluid, tracheal fluid, ascites, cystic tumor fluid, amniotic fluid, and combinations thereof, which are obtained from the body, and may include a culture cell medium, and is not limited thereto as long as the sample is any sample that secretes exosomes. Here, the medium may contain Fetal Bovine Serum (FBS), but is not limited thereto, and may be used with commercially available CellCor^{™}CD MSC from Xcell Therapeutics, MesenCult^{™}plus from STEMCELL technology, eproGrow^{™}hMSC (Mesenchymal Stem Cell) Media from Peprotech, MSC Nutristem ^{®} series from BI/Satorius, StemMACS^{™} from Miltenyi biotec, RoosterNourish^{™} from Roosterbio, StemXVivo^{™} from R&D systems, Cellartis^{®} MSC XF from Takara Bio, PRIME-XV XSFM or PRIME-XV SFM from Irvine Science/FUJI, StemFit for MSC from Ajinomoto, TheraPEAK^{™} from Lonza, CnT-PR-MSC-XF from Cellntec, etc.

In an example, blood refers to a body fluid that supplies oxygen and nutrients to cells in the body and recovers and carries carbon dioxide and waste products produced by cell metabolism. The plasma is a liquid component constituting the blood, and serves as a solvent in which various types of organic and inorganic substances, including proteins, are dissolved. The serum or fluid is a component of the blood, and refers to a yellow liquid component remaining in plasma after fibrinogen has been removed.

The method for improving the exosome productivity of a specific exemplary embodiment of the present invention is characterized by being able to achieve increased production efficiency and yield, and more specifically, by treating high-pressure oxygen by changing an environmental condition between a general culture condition and a favorable condition, such as a pressure condition inside an incubator, an oxygen concentration condition, a carbon dioxide concentration condition, a nitrogen concentration condition, an air composition condition, a wavelength condition of a light source, and a temperature condition during the stem cell culture process in order to increase the production efficiency and yield of exosomes.

In a specific exemplary embodiment, the method includes culturing a sample containing cells in a first pressure environment; and culturing the sample in a second pressure environment higher than the first pressure, and the second pressure condition is created by high-pressure oxygen to increase the production efficiency and yield of exosomes, but is not limited thereto.

More specifically, the method may further include extracting exosomes from the sample, but is not limited thereto.

More specifically, the cells may be stem cells, but are not limited thereto.

More specifically, the culturing in the first pressure environment and the culturing in the second pressure environment may be repeated two or more times, but are not limited thereto.

More specifically, the second pressure may be 1 to 5 ATA, but is not limited thereto.

More specifically, the culturing of the sample in the second pressure environment may be performed for 60 to 120 minutes, but is not limited thereto.

As used in the present disclosure, the term 'productivity' and 'yield' refer to the amount of exosomes that may be obtained from a predetermined sample, and means being measured through a general quantification method including the experimental method described in the example.

In a specific exemplary embodiment, in the method for improving the production efficiency and yield of exosomes, the production efficiency and yield may be defined as the number of exosome particles per mL of an exosome culture solution.

Hereinafter, the present invention will be described in more detail through Examples. However, these Examples are only illustrative the present invention, and the scope of the present invention is not limited to these Examples.

### Advantageous Effects

According to the example of the present invention, the method for increasing the exosome productivity has effects of increasing the production efficiency and yield by culturing the cells treated with high-pressure oxygen under an environment condition between a relatively harsh condition and a relatively favorable condition, for example, a favorable condition in a stem cell culture process.

More specifically, the high-pressure oxygen as the favorable condition may be treated 1 to 5 times for 60 to 120 minutes under 1 to 5 ATA conditions, preferably 3 times for 90 minutes under a 3ATA condition. As a result, it can be seen that the production efficiency and yield of exosomes are increased.

The effects according to the present invention are not limited to the contents exemplified above, and further various effects are included in the present specification.

### Description of Drawings

FIG. 1 is a diagram illustrating a high-temperature oxygen treatment method according to an example of the present invention.
FIG. 2 is a diagram illustrating the size and concentration distribution of exosomes according to a result of nanoparticle tracking analysis (NTA) in a control group A0.
FIG. 3 is a diagram illustrating the size and concentration distribution of exosomes according to a result of nanoparticle tracking analysis (NTA) in an experimental group A1.
FIG. 4 is a diagram illustrating the size and concentration distribution of exosomes according to a result of nanoparticle tracking analysis (NTA) in an experimental group A2.
FIG. 5 is a diagram illustrating the size and concentration distribution of exosomes according to a result of nanoparticle tracking analysis (NTA) in an experimental group A3.

### Best Mode of the Invention

Although the examples of the present invention have been described in detail with reference to the accompanying drawings, the present invention is not limited thereto and may be embodied in many different forms without departing from the technical concept of the present invention.

Therefore, the examples of the present invention are provided for illustrative purposes only but not intended to limit the technical concept of the present invention. The scope of the technical concept of the present invention is not limited thereto. Therefore, it should be appreciated that the aforementioned examples are illustrative in all aspects and are not restricted. The protective scope of the present invention should be construed on the basis of the appended claims, and all the technical ideas in the equivalent scope thereof should be construed as falling within the scope of the present invention.

Hereinafter, the present invention will be described in more detail through Examples. However, these Examples are intended to illustrate one or more exemplary embodiments, and the scope of the present invention is not limited to these Examples.

### Example 1. Cell culture

For exosome extraction, 70 cc of fat was suctioned from the lower abdomen of a healthy adult man aged 30 to 40 years, and the stromal vascular fraction (SVF) was extracted and then frozen in a - 196°C nitrogen tank at atmospheric pressure.

Thereafter, the frozen SVF was thawed and inoculated into a flask using a DMEM (Dulbecco's Modified Eagle Medium) culture medium containing fetal bovine serum (FBS), 100 units/mL penicillin, and 100 µg/mL streptomycin, cultured for 7 days in an incubator at 37°C, Air, and 5% CO₂, and further cultured for 18 days under the same conditions to secure sufficient stem cells.

### Example 2. Treatment of high-pressure oxygen

The cells cultured in Example 1 were divided into four groups A0, A1, A2, and A3 and treated with high-pressure oxygen by varying the number of times as follows.

Each sample was dispensed so that 10⁵ cells were per well (10 ml), and the control and experimental groups were added in two wells, respectively, and the experiment was performed.

The treatment with high-pressure oxygen was performed by culturing the cells in a high-pressure oxygen chamber at 3 ATA for 90 minutes, and when the treatment with high-pressure oxygen was not performed, the cells were cultured in a general incubator under 37°C, Air, and 5% CO₂ conditions.

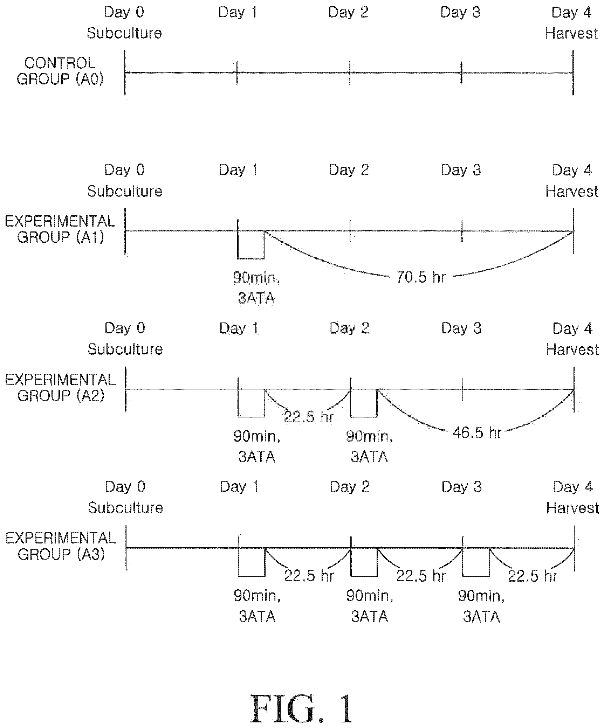

More specifically, as illustrated in FIG. 1, the control group A0 was cultured for 4 days in a general incubator without treatment with high-pressure oxygen. The experimental group A1 was cultured once in a high-pressure oxygen chamber on Day 1 of culture, and then cultured in a general incubator for 70.5 hours, the experimental group A2 was cultured in a high-pressure oxygen chamber total twice, once each on Day 1 and Day 2 of culture, and then cultured [81]in a general incubator for 46.5 hours, and the experimental group A3 was cultured in a high-pressure oxygen chamber total three times, once each on Day 1, Day 2, and Day 3 of culture, and then further cultured in a general incubator for 22.5 hours. Thereafter, on Day 4 of culture, the control group A0 and the experimental groups A1 to A3 were all collected, and a total of 8-well culture solutions were frozen in a - 196°C nitrogen tank.

### Example 3. Exosome extraction and characteristic analysis

In order to measure exosome productivity and yield from the cells cultured in Example 1 and the stem cells treated with high-temperature oxygen in Example 2, the culture solutions were obtained from each of the control and experimental groups, and then exosomes were isolated using an Aqueous Two-Phase System (ATPS) method.

The isolated exosomes were subjected to nanoparticle tracking analysis to measure the particle size and concentration.

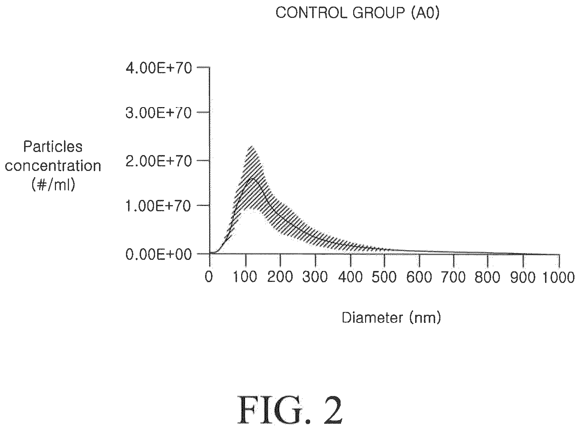

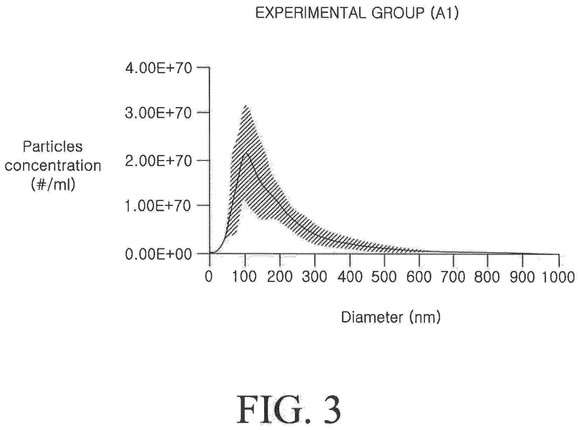

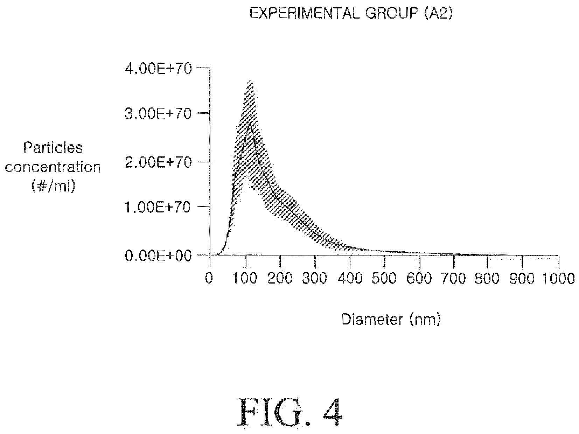

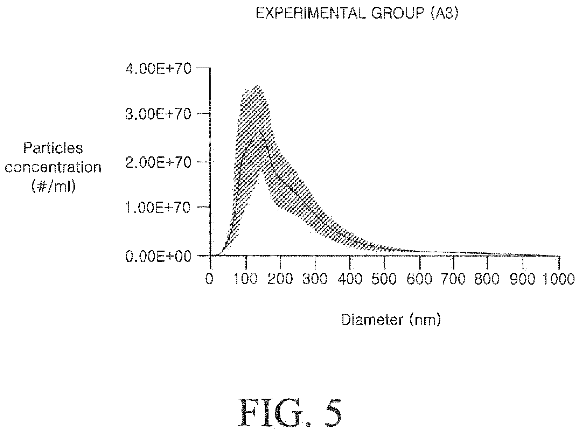

As a result, as illustrated in FIGS. 2 to 5 and Table 1, it was confirmed that the isolated exosomes were distributed in a size range of approximately 200 nm (the conventional size range of exosomes), and the particle size distribution was generally uniform to satisfy the size and distribution characteristics of exosomes. In addition, as shown in Table 1, it was confirmed that in the case of A1 to A3 treated with high-pressure oxygen, the number concentration of exosomes increased as the number of high-pressure oxygen treatments increased, and thus the exosome productivity was improved.

**[Table 1]**

| **Kelly ratio** | 1.765 | | | | | | | | | |
|---|---|---|---|---|---|---|---|---|---|---|
| **Unit** | | #/ml | | | | g | g | # | $/L | |
| **Sample** | Diluti or | **Exosome number concentration** | Total particl e # (STD) | Average diameter [nm] | Aver age diam eter (STD) | **Initial volume** | **Final volume** | **Exosome number after isolation** | **Number per 1 L** | **Number error per 1 L** |
| A0 | 500 | 4.61 E+ 09 | 9.06E+ 08 | 209.4 | 19.6 | 19.221 | 0.171 | 7.88.E +08 | 4.10.E +10 | 8.06.E +09 |
| A1 | 500 | 6.15E+ 09 | 1.67E+ 09 | 216.0 | 27.6 | 18.484 | 0.238 | 1.46.E +09 | 7.92.E +10 | 2.15.E +10 |
| A2 | 500 | 7.01 E+ 09 | 1.10E+ 09 | 199.0 | 20.6 | 18.505 | 0.176 | 1.23.E +09 | 6.66.E +10 | 1.05.E +10 |
| A3 | 500 | 8.98E+ 09 | 2.27E+ 09 | 228.4 | 21.4 | 18.495 | 0.211 | 1.90.E +09 | 1.02.E +11 | 2.59.E +10 |

In addition, flow cytometry was performed on the isolated exosomes to confirm the presence of CD9, CD63, and CD81 markers.

Since CD9, CD63, and CD81 are representative positive markers of exosomes, the contents of CD9, CD63, and CD81 and the content of exosomes have linearity to each other, and an increase in the contents of CD9, CD63, and CD81 means a proportional increase in the content of exosomes.

Specifically, in order to isolate exosomes positive for CD81, an exosome-human CD81 isolation/detection kit (ThermoFisher Scientific) was used according to the manufacturer's instructions, markers were stained using PE-Mouse anti-human CD9 antibody (PE-Mouse anti-human CD9; BD Biosciences), PE-Mouse anti-human CD63 antibody (PE-Mouse anti-human CD63; BD Biosciences), and PE-Mouse anti-human CD81 antibody (PE-mouse anti-human CD81; BD Biosciences), and then analyzed using a flow cytometer (ACEA Biosciences). As a result, as shown in FIG. 6, it was confirmed that the exosomes isolated in Example 2 were positive for the positive markers CD9, CD63, and CD81. These results indicate that the isolated exosomes satisfy the specific marker characteristics that are necessarily present in exosomes.

Therefore, it was confirmed that the exosomes were able to be effectively obtained and mass-produced with high yield when using the present invention.

Meanwhile, the above-described experiment was conducted in a general incubator and an incubator capable of high-pressure oxygen treatment, but is not limited thereto, and the method according to the present invention may be performed in a bioreactor capable of controlling pressure internally. That is, the method of the present invention may be performed by alternately culturing the sample between incubators having two pressure conditions, or the method of the present invention may also be performed by changing the pressure conditions within a space in the bioreactor.

Although the examples of the present invention have been described in detail with reference to the accompanying drawings, the present invention is not limited thereto and may be embodied in many different forms without departing from the technical concept of the present invention. Therefore, the examples disclosed in the present invention are intended not to limit the technical spirit of the present invention but to describe the present invention and the scope of the technical spirit of the present invention is not limited by these examples. Therefore, it should be understood that the above-described examples are illustrative in all aspects and do not limit the present invention. The protective scope of the present invention should be construed on the basis of the appended claims, and all the technical ideas in the equivalent scope thereof should be construed as falling within the scope of the present invention.

## Claims

1. A method for improving exosome productivity comprising:
culturing a sample containing cells in a first pressure environment; and
culturing the sample in a second pressure environment higher than the first pressure,
wherein the second pressure condition is created by high-pressure oxygen.

2. The method for improving exosome productivity of claim 1, further comprising:
extracting exosomes from the sample.

3. The method for improving exosome productivity of claim 1, wherein the cells are stem cells.

4. The method for improving exosome productivity of claim 1, wherein the culturing in the first pressure environment and the culturing in the second pressure environment are repeated two or more times.

5. The method for improving exosome productivity of claim 1, wherein the second pressure is 1 to 5 ATA.

6. The method for improving exosome productivity of claim 1, wherein the culturing of the sample in the second pressure environment is performed for 60 to 120 minutes.

7. A composition comprising exosomes having the number concentration of 5 × 10⁹ or more per 1 ml.
